# EUROPEAN PATENT SPECIFICATION

(11) **EP 3 192 508 B1**
(45) Date of publication and mention of the grant of the patent: **08.05.2019**
(21) Application number: 14901531.5
(22) Date of filing: 12.09.2014
(51) Int. Cl.: A61K 31/4468, A61K 9/08, A61K 9/19, A61K 47/22, A61K 47/24, A61K 9/00, A61K 47/26

(54) **REMIFENTANIL INJECTION CONTAINING COLORING AGENT**
REMIFENTANILINJEKTION MIT FARBSTOFF
INJECTION DE RÉMIFENTANIL CONTENANT UN AGENT COLORANT

(43) Date of publication of application: 19.07.2017
(73) Proprietor: Terumo Kabushiki Kaisha, Tokyo 151-0072 (JP)
(72) Inventor: HORIUCHI, Aiko, Kanagawa 259-0151 (JP); HOJO, Tomoyuki, Kanagawa 259-0151 (JP)
(74) Representative: Casalonga
(86) International application number: PCT/JP2014/074228
(87) International publication number: WO 2016/038735

(56) References cited:
- JP-A- 2002 104 996
- JP-A- 2010 528 001
- US-A1- 2010 261 747
- US-A1- 2014 179 740
- US-B2- 8 426 467
- MASARU TANNO: 'Aruchiba TM no 'Tokiwasure' Taisaku' THE JOURNAL OF JAPAN SOCIETY FOR CLINICAL ANESTHESIA, [Online] vol. 31, no. 7, November 2011, page 1020, XP055358395 Retrieved from the Internet: <URL:HTTPS://WWW.JSTAGE.JST.GO.JP/ARTICLE/J JSCA/31/7/31_7_1020/_PDF>
- 'Janssen Pharmaceutical Kabushiki Kaisha' ARUCHIBA R JOCHUYO 2MG, ARUCHIBA R JOCHUYO 5MG TENPU BUNSHO August 2009, XP008184886

## Description

### TECHNICAL FIELD

The present invention relates to a remifentanil injection formulation. More particularly, the present invention relates to a remifentanil injection formulation containing a colorant.

### BACKGROUND ART

An opioid analgesic has a strong analgesic action and stabilizes stress reactions of circulation dynamics caused by surgical invasion. The opioid analgesic satisfies the elements of anesthesia having sedative, analgesic, and muscle relaxant actions caused by a surgical operation and keeps stresses applied to a patient during the surgical operation to the minimum, thereby restraining the extent of the stress reactions of the circulation dynamics such as the rise of a blood pressure, tachycardia, and the like caused by the surgical stresses to the minimum. From this standpoint, the opioid analgesic is suitable as an analgesic in performing general anesthesia in that the opioid analgesic surely gives ease and comfort to a patient for her/his pain after the surgical operation is performed.

Fentanyl was used as the opioid analgesic at an anesthetizing time for the past several decades. Sufentanil and alfentanil were approved as next-generation analgesics in western countries and used as the opioid analgesic at the anesthetizing time. Under such circumstances, remifentanil was approved in October of 2006 in Japan and started to be sold from January of 2007. Currently the remifentanil is used as the opioid analgesic when general anesthesia is performed.

Fentanyl is metabolized into active and nonactive metabolites in a liver, and only 10% of the metabolites is discharged from a kidney. Thus, in a case where the fentanyl is continuously medicated, it is accumulated in a body and its action remains after the medication finishes. As a result, disease symptoms such as late-onset respiratory depression, arousal delay, nausea, and vomiting appear. On the other hand, remifentanil is common with the fentanyl in that the remifentanil is a selective p-opioid receptor agonist, but is different from the fentanyl in that the remifentanil is rapidly hydrolyzed by nonspecific esterase in blood and a living tissue and becomes the nonactive metabolite which is discharged from the kidney. The remifentanil is prompt in the appearance and disappearance of its analgesic action and does not have the property of accumulating in the body and further, is capable of easily controlling pain relief according to invasive stimulation. Because the remifentanil can be used for patients having a disorder in renal and liver functions without adjusting its capacity, the remifentanil is currently used in combination with an inhalation anesthetic and an intravenous anesthetic as an analgesic in introducing and maintaining general anesthesia (see nonpatent documents 1 and 2).

The remifentanil is soluble in water, but an aqueous solution thereof is unstable and cannot be stored for a long term. Therefore, currently lyophilized powder of the remifentanil is distributed and sold as a remifentanil injection formulation by accommodating it in containers such as a vial container.

A method of preparing a remifentanil injection solution is adopted to administer the remifentanil injection solution to patients at an anesthetizing time. The method includes the process of preparing a concentrate solution of the remifentanil by injecting a small amount of a dissolving solution (water, saline or 5% glucose injection solution) to a container such as a vial container accommodating a lyophilized remifentanil formulation to dissolve the remifentanil, the process of collecting the concentrate solution from the container, and the process of mixing a diluting solution (saline, 5% glucose injection solution) with the concentrate solution to prepare a final remifentanil injection solution to be injected to patients (see left-hand column of page 1655 of nonpatent document 1).

Not only the remifentanil, but also a large number of medical agents are distributed and sold in the form of a lyophilized formulation and liquefied by using the dissolving and diluting solutions when the medical agents are administered to patients.

Most of anesthetics are distributed and sold in the form of a liquid injection solution formulation or a gaseous formulation. It is rare that anesthetics are distributed and sold in the form of the lyophilized formulation. Thus, there was a case in which mistakenly believing that the remifentanil injection formulation was originally in a liquid state like normal anesthetics, medical workers working at a medical front forgot to liquefy the lyophilized remifentanil injection formulation by using dissolving and diluting solutions and administered the dissolving and diluting solutions to patients by mistake before the dissolving and diluting solutions were mixed with the remifentanil injection formulation. To avoid the occurrence of such a trouble, it is necessary for medical workers working at the medical front to confirm that the injection solution to be administered to patients at an anesthetizing time contains the remifentanil.

But the remifentanil injection solution prepared by adding the dissolving and diluting solutions to the remifentanil and the dissolving and diluting solutions which have not been mixed with the remifentanil are colorless. Thus, it is difficult to distinguish the remifentanil injection solution from the dissolving and diluting solutions. Therefore, medical workers working at the medical front take considerable time and effort to check whether the injection solutions to be administered to patients contain the remifentanil and have been prepared by adding the dissolving and diluting solutions to the remifentanil by inquiring operators in charge about the case and check whether vacant containers from which the remifentanil has been taken out are left at the medical front. Thus, medical workers working at the medical front have much burden.

To prevent the occurrence of medical malpractice accompanied by medication errors, an art of mixing a pigment such as food blue No. 1 with a medical agent such as thrombin having a hemostatic action is known. The art was developed to prevent a hemostatic agent such as the thrombin from being injected to human bodies by taking the hemostatic agent such as the thrombin for an injection medicine (patent document 1).

The art of the patent document 1 was developed to prevent a medical agent from being erroneously used, i.e., prevent the topical hemostatic agent such as the thrombin which is to be endoscopically sprayed from being injected to human bodies. The art of the patent document 1 is not intended to check whether an injection solution contains a predetermined medical agent. In the patent document 1 relating to the art of the coloring of the hemostatic agent such as the thrombin, neither disclosure nor suggestion was made to the effect that the art of the patent reference 1 is applicable to the remifentanil injection formulation greatly different from the hemostatic agent such as the thrombin in terms of the kind, property, action, and usage thereof.

### PRIOR ART DOCUMENTS

### PATENT DOCUMENTS

Patent document 1: Japanese Patent Application Laid-Open Publication No. 2002-104996

### NONPATENT DOCUMENT

Nonpatent document 1: "Anesthesia 21 Century" vol. 9, No.2-28, P. 1652-1665 (2007)
Nonpatent document 2: Medical Journal of Kagoshima City, vol. 46, No. 6, p. 12 (2007)

### SUMMARY OF THE INVENTION

### PROBLEMS TO BE SOLVED BY THE INVENTION

It is an object of the present invention to provide a remifentanil injection formulation which prevents a trouble of injecting dissolving and diluting solutions to patients because workers working at a medical front forgets to dissolve and dilute the remifentanil injection formulation with the dissolving and diluting solutions and allows workers at the medical front to easily and reliably confirm that an injection solution to be injected to patients contains the remifentanil and has been prepared by mixing the dissolving and diluting solutions with the remifentanil injection formulation.

It is another object of the present invention to provide a remifentanil injection formulation which prevents a remifentanil-containing injection solution prepared by dissolving and diluting the remifentanil injection formulation with the dissolving and diluting solutions from adversely affecting the operation of a vital sign monitor such as a pulse oximeter mounted on a patient to monitor the state of the patient at an anesthetizing time.

It is still another object of the present invention to provide a remifentanil injection formulation which allows the remifentanil to hardly decompose and denature and to be maintained at a high survival rate even when the remifentanil injection formulation is stored for a long term at a normal temperature and is thus excellent in its long-term storage stability.

### MEANS FOR SOLVING THE PROBLEMS

In continuous researches made by the present inventors to achieve the above-described objects, they have come to realize that by containing a colorant in the remifentanil injection formulation, it is possible to prevent a trouble of injecting dissolving and diluting solutions which have not been mixed with remifentanil to patients by mistaking the dissolving and diluting solutions for the remifentanil injection solution formulation.

Based on the above-described finding, they have repeatedly made a lot of experiments by using various colorants regarded as being safe for living bodies. As a result, they have found that of a large number of colorants, colorants selected from among cyanocobalamin, indocyanine green, and indigocarmine suit the object of the present invention.

A colored remifentanil solution (remifentanil injection solution) is obtained by mixing the dissolving and diluting solutions with the remifentanil injection formulation prepared by mixing a specific colorant selected from among the cyanocobalamin, the indocyanine green, and the indigocarmine with the remifentanil and/or physiologically acceptable remifentanil salts of the remifentanil. Thus, the remifentanil solution is distinctly distinguished from the colorless dissolving and diluting solutions. Consequently, it is possible to securely prevent the occurrence of the trouble of injecting the dissolving and diluting solutions to patients by mistake.

The present inventors have also found that any of the cyanocobalamin, the indocyanine green, and the indigocarmine does not decompose the remifentanil (salts) or deteriorate the quality thereof and that the remifentanil injection formulation obtained by mixing the cyanocobalamin, the indocyanine green or the indigocarmine with the remifentanil (salts) has long-term storage stability equivalent to that of the remifentanil injection formulation not containing these colorants.

The present inventors have also found that the cyanocobalamin, the indocyanine green or the indigocarmine contained in the remifentanil injection formulation does not deteriorate the anesthetic and analgesic functions of the remifentanil injection formulation. The present inventors have completed the present invention based on the above-described findings.

The present invention provides:
(1) A remifentanil injection formulation containing remifentanil and/or physiologically acceptable salts of the remifentanil and a colorant characterized in that said colorant is cyanocobalamin.

In the present invention,
(2) In the remifentanil injection formulation according to the above (1), the colorant is the cyanocobalamin.
(3) In the remifentanil injection formulation according to the above (1) or the above (2), the colorant is contained at a rate of 0.02 to 0.30mg per 1mg of the remifentanil.
(4) The remifentanil injection formulation according to any one of the above (1) through the above (3) is liquid.
(5) In the remifentanil injection formulation according to the above (4), as a content of the remifentanil, the remifentanil and/or the physiologically acceptable salts of the remifentanil is contained at a rate of 1 to 10mg in 1mL of the remifentanil injection solution formulation.
(6) The remifentanil injection formulation according to any one of the above (1) through the above (3) is dry.
(7) A kit formulation having a container accommodating a remifentanil injection formulation according to any one of the above (1) through the above (6) and a container accommodating a dissolving solution and/or a container accommodating a diluting solution.

### EFFECT OF THE INVENTION

The remifentanil injection solution prepared by dissolving and diluting the remifentanil injection formulation of the present invention with the dissolving and diluting solutions assumes the color derived from the colorant cyanocobalamin and thus can be distinctly distinguished from the colorless dissolving and diluting solutions. Therefore, by using the remifentanil injection formulation of the present invention containing the colorant, it is possible to prevent the occurrence of medical malpractice of erroneously injecting the dissolving and diluting solutions which have not been mixed with the remifentanil injection formulation and do not contain the remifentanil to patients.

The remifentanil injection formulation of the present invention containing the colorant cyanocobalamin, and the indigocarmine maintains the excellent anesthesia and analgesic functions to the same extent as that when the remifentanil injection formulation does not contain the colorant.

Any colorant selected from among the cyanocobalamin, the indocyanine green, and the indigocarmine contained in the remifentanil injection formulation does not adversely affect the stability of the remifentanil. Therefore, the remifentanil injection formulation containing the colorant selected from among the cyanocobalamin, the indocyanine green, and the indigocarmine is excellent in its long-term storage stability as with the remifentanil injection formulation not containing the colorant.

The remifentanil injection formulation of the present invention containing the cyanocobalamin is excellent in its long-term storage stability both when the remifentanil injection formulation is dry and when the remifentanil injection formulation is the liquid injection solution. Thus, even though the remifentanil injection formulation is stored for a long term at the normal temperature, the remifentanil hardly decomposes and denatures.

The remifentanil injection formulation of the present invention containing the cyanocobalamin does not change or fade in its color after it is stored for a long term.

An injection solution prepared by adding the dissolving solution and/or the diluting solution to the remifentanil injection formulation of the present invention containing the cyanocobalamin does not adversely affect the operation of a vital sign monitor such as a pulse oximeter for monitoring the state of a patient at an anesthetizing time.

### MODE FOR CARRYING OUT THE INVENTION

The present invention is described in detail below.

"Remifentanil and/or physiologically acceptable salts thereof" (may be referred to as "remifentanil (salts)") for use in the remifentanil injection formulation of the present invention are also known as methyl4-(methoxycarbonyl)-4-[(1-oxopropyl)phenylamino]piperidine-1-propionic acid methyl ester.

In the present invention, as the remifentanil (salts), it is possible to use one or not less than two of remifentanil itself not in the form of salts and salt selected from among the physiologically acceptable salts thereof.

As the physiologically acceptable salts of the remifentanil, it is possible to exemplify hydrochloride, hydrobromate, sulfate, sulfonate, phosphate, tartrate, formate, acetate, propionate, benzoate, oxalate, succinate, citrate, glutamate, fumarate, aspartate, glutarate, stearate, butyrate, malonate, and lactate. In the present invention, it is possible to use one or not less than two kinds of the above-described salts of the remifentanil.

Of the above-described salts, in the present invention, the remifentanil and/or the remifentanil hydrochloride are favorably used as the remifentanil (salts). The remifentanil hydrochloride is more favorably used in terms of its availability.

The remifentanil injection formulation of the present invention contains cyanocobalamin.

The cyanocobalamin is a kind of water-soluble vitamins called vitamin B₁₂ and is a complex of cobalt having a structure of a corrin ring similar to the structure of porphyrin and the structure of nucleotide. The cyanocobalamin assumes red or pink and has been used to cure eyestrain, peripheral nerve disorder, and megaloblastic anemia.

The indocyanine green is a non-poisonous green pigment insusceptible to chemical and optical changes and has been used for a circulatory function test and a liver function test.

The indigocarmine is an artificial colorant classified into an indigoid edible tar dyes and is a granular or powdery solid having a dark purple blue color at a normal temperature. As a medical use, the indigocarmine is used for chromoendoscopy.

Any colorant selected from among the cyanocobalamin, the indocyanine green, and the indigocarmine contained in the remifentanil injection formulation does not decompose the remifentanil (salts) or deteriorate the quality thereof. Therefore, the remifentanil injection formulation containing the colorant selected from among the cyanocobalamin, the indocyanine green, and the indigocarmine does not deteriorate in its anesthesia and analgesic functions and storage stability.

Considering the properties of the above-described colorants, any colorant selected from among the cyanocobalamin, the indocyanine green, and the indigocarmine can be effectively used.

Of these colorants, the cyanocobalamin does not fade in its color or discolor when it is stored for a long term and is capable of maintaining its initial color tone in a case where the cyanocobalamin is contained in solid and liquid remifentanil injection formulations. The anesthetic remifentanil injection solution prepared by dissolving and diluting the remifentanil injection formulation containing the cyanocobalamin with the dissolving and diluting solutions does not absorb light, having a measuring wavelength of 660nm, emitted from a pulse oximeter or adversely affect the operation of the pulse oximeter at an anesthetizing time. Therefore, the cyanocobalamin is used more favorably than the other colorants.

In a case where the indocyanine green or the indigocarmine is used, to prevent the remifentanil injection formulation from fading or changing in its color during storage, it is desirable to produce the remifentanil injection formulation in the form of lyophilized powder or lyophilized fine particles.

As colorants for medical agents, riboflavin phosphate ester sodium, uranine (another name: sodium fluorescein), and the like are known. The present inventors conducted a stability test of the remifentanil injection formulations containing these colorants. As a result, these colorants greatly lowered the survival rate of the remifentanil. Thus, these colorants cannot be used as the colorant for the remifentanil injection formulation.

It is preferable to set the content of the colorant in the remifentanil injection formulation to 0.02 to 0.30mg per 1mg of the remifentanil (remifentanil not in the form of salts). By setting the content of the colorant to the above-described range, it is possible to distinctly distinguish the color tone of the remifentanil injection solution for anesthesia which is obtained by dissolving and diluting the remifentanil injection formulation with the dissolving and diluting solutions from the color tones of the dissolving and diluting solutions which have not been mixed with the remifentanil injection formulation and do not contain the remifentanil by a visual observation. Thus, it is possible to prevent the occurrence of a trouble of injecting the dissolving and diluting solutions not containing the remifentanil to a patient at an anesthetizing time.

More specifically, in a case where the cyanocobalamin is used as the colorant for the remifentanil injection formulation, it is more favorable for the remifentanil injection formulation to contain 0.05 to 0.30mg of the cyanocobalamin and most favorable to contain 0.10 to 0.20mg thereof per 1mg of the remifentanil contained in the remifentanil injection formulation.

In a case where the indocyanine green is used as the colorant for the remifentanil injection formulation, it is more favorable for the remifentanil injection formulation to contain 0.05 to 0.15mg of the indocyanine green and most favorable to contain 0.05 to 0.10mg thereof per 1mg of the remifentanil contained in the remifentanil injection formulation.

In a case where the indigocarmine is used as the colorant for the remifentanil injection formulation, it is more favorable for the remifentanil injection formulation to contain 0.02 to 0.10mg of the indocyanine green and most favorable to contain 0.04 to 0.10mg thereof per 1mg of the remifentanil contained in the remifentanil injection formulation.

The remifentanil injection formulation of the present invention containing the cyanocobalamin as the colorant therefor may be the liquid remifentanil injection solution formulation or a dry and solid remifentanil injection formulation.

In a case where the remifentanil injection formulation containing the indocyanine green or the indigocarmine as the colorant therefor is produced as a solution formulation, the remifentanil injection formulation may change or fade in its color during storage. Thus, it is desirable to produce the remifentanil injection formulation as the dry and solid remifentanil injection formulation.

In a case where the remifentanil injection formulation of the present invention is the liquid remifentanil injection solution formulation containing the cyanocobalamin, it is not necessary to perform a dissolving operation of dissolving the remifentanil formulation with a small amount of the dissolving solution. The remifentanil injection solution can be easily prepared by performing an operation of merely mixing the remifentanil injection solution formulation accommodated inside a container with the diluting solution.

In a case where the remifentanil injection formulation containing the colorant selected from among the cyanocobalamin, the indocyanine green, and the indigocarmine is the dry and solid formulation, the remifentanil injection formulation is preferably a lyophilized granular or powdery formulation in consideration of its solubility in the dissolving and diluting solutions.

In a case where the remifentanil injection formulation of the present invention is the dry and solid formulation, the remifentanil injection solution to be used at an anesthetizing time is prepared by adding a small amount of the dissolving solution to the remifentanil injection formulation accommodated inside a container to dissolve it and thereafter adding the diluting solution to dilute it.

(I) In a case where the remifentanil injection formulation of the present invention is the liquid remifentanil injection formulation containing the cyanocobalamin, to allow the remifentanil (salts) to dissolve well in the injection solution formulation and to be excellent in its long-term storage stability,
(I) it is preferable for the remifentanil injection solution formulation [hereinafter may be referred to as "remifentanil injection solution formulation (I)"] to contain the remifentanil (salts), the cyanocobalamin, water (pharmaceutically, pharmacologically, and physiologically acceptable water such as distilled water, normal water, purified water, sterile purified water, water for injection, and distilled water for injection), and a stabilizer in such a way that the content of the water per 1mg of the remifentanil is set to 0.007 to 0.025mL, favorably 0.007 to 0.02mL, more favorably 0.008 to 0.015mL, and especially favorably 0.009 to 0.015mL or
(II) it is preferable for the remifentanil injection solution formulation [hereinafter may be referred to as "remifentanil injection solution formulation (II)"] to contain the remifentanil (salts), the cyanocobalamin, ethanol, and the stabilizer in such a way that the content of the ethanol per 1mg of the remifentanil is set to 0.10 to 0.50mL, favorably 0.10 to 0.35mL, and especially favorably 0.12 to 0.30mL.

In the remifentanil injection solution formulation (I), by setting the content of the water per 1mg of the remifentanil to the above-described range, the remifentanil (salts) dissolves preferably in the water and is stabilized as it is by the stabilizer. Thus, even after the remifentanil injection solution formulation (I) is stored at the room temperature for a long term, the remifentanil is prevented from denaturing, decomposing, and precipitating and thus dissolves and is maintained as it is in the solution at a high survival rate. Therefore, the remifentanil injection solution formulation (I) is excellent in its long-term storage stability.

In a case where the content rate of the water per 1mg of the remifentanil is less than 0.007mL, the remifentanil (salts) does not sufficiently dissolve in the water and is liable to precipitate during the storage of the remifentanil injection solution formulation even though it has dissolved in the water. On the other hand, in a case where the content rate of the water per 1mg of the remifentanil exceeds 0.025mL, there is no problem in terms of the solubility of the remifentanil (salts) in the water. But in a case where the remifentanil injection solution formulation is stored for a long term, the remifentanil (salts) denatures and decomposes and thus its survival rate decreases.

In the present specification, "per 1mg of the remifentanil" means "per 1mg of the remifentanil not in the form of salts".

In a case where the remifentanil injection solution formulation (I) contains not less than two kinds of the remifentanil (salts), the above-described content rate of the water means its content rate per the total amount of 1mg of the remifentanil.

In the present specification, the content rate (mL) of the water per 1mg of the remifentanil in the remifentanil injection solution formulation (I) means the content rate (mL) of the water at 20 degrees C.

In the remifentanil injection solution formulation (II), by setting the content of the ethanol per 1mg of the remifentanil to the above-described range, the remifentanil (salts) dissolves well in the ethanol and is stabilized by the stabilizer contained in the remifentanil injection solution formulation (II). Thereby even after the remifentanil injection solution formulation (II) is stored for a long term at the room temperature, the remifentanil hardly denatures and decomposes and is maintained in the solution at a high survival rate and is thus excellent in its long-term storage stability.

In a case where the content rate of the ethanol per 1mg of the remifentanil is below 0.1mL, the remifentanil (salts) does not sufficiently dissolve in the ethanol and is liable to precipitate during the storage of the remifentanil injection solution formulation (II) even in a case where the remifentanil (salts) has dissolved therein. On the other hand, in a case where the content rate of the ethanol per 1mg of the remifentanil exceeds 0.50mL, there is no problem in terms of the solubility of the remifentanil (salts) in the ethanol. But even though the remifentanil injection solution formulation (II) contains the stabilizer, the remifentanil (salts) denatures and decomposes when the remifentanil (salts) is stored for a long term and consequently its survival rate decreases.

In the present specification, the content rate (mL) of the ethanol per 1mg of the remifentanil in the remifentanil injection solution formulation (II) means the content rate (mL) of the ethanol at 20 degrees C.

In a case where the remifentanil injection solution formulation (II) contains not less than two kinds of the remifentanil (salts), the above-described content rate of the ethanol means its content rate per the total amount of 1mg of the remifentanil.

The stabilizer is used for the remifentanil injection solution formulations (I) and (II) of the present invention to dissolve the remifentanil (salts) stably in the water or in the ethanol. As the stabilizer, liquid polyhydric alcohol physiologically acceptable can be preferably used. As concrete examples of the stabilizer, it is possible to exemplify liquid polyethylene glycol, liquid propylene glycol, and the like. Of these stabilizers, the liquid polyethylene glycol having a molecular weight of 200 to 600 daltons is more favorably used.

As an example of the liquid polyethylene glycol having the molecular weight in the range of 200 to 600 daltons, macrogol 400, macrogol 200, and the like are exemplified.

In the remifentanil injection solution formulation (I), the content ratio between the water and the stabilizer is set to favorably 2:23 to 2:198, more favorably 2:23 to 2:98, and most favorably 2:48 to 2:98 in a volume ratio.

In the remifentanil injection solution formulation (II) of the present invention, the content ratio between the ethanol and the stabilizer is set to preferably 3:2 to 3:7 in a volume ratio.

By setting the volume ratio between the water and the stabilizer in the remifentanil injection solution formulation (I) and the volume ratio between the ethanol and the stabilizer in the remifentanil injection solution formulation (II) to the above-described range respectively, the amount of the stabilizer in the remifentanil injection solution formulation (I) and that of the stabilizer in the remifentanil injection solution formulation (II) are sufficient. Thus, the osmotic pressure falls within a range in which intravenous injection can be accomplished and further, the long-term storage stability of the remifentanil injection solution formulation can be secured.

In the present specification, the content ratio (volume ratio) between the water and the stabilizer in the remifentanil injection solution formulation (I) means the content ratio (volume ratio) therebetween at 20 degrees C. Similarly, the content ratio (volume ratio) between the ethanol and the stabilizer in the remifentanil injection solution formulation (II) means the content ratio (volume ratio) therebetween at 20 degrees C.

In a case where the content ratio between the water and the stabilizer in the remifentanil injection solution formulation (I) departs from the above-described range and the content ratio of the stabilizer is too low, it is difficult to obtain the remifentanil injection solution formulation in which the remifentanil (salts) has stably dissolved in the water. On the other hand, in a case where the content ratio of the stabilizer in the remifentanil injection solution formulation (II) is too high, an osmotic pressure is liable to become high.

In a case where the content ratio between the ethanol and the stabilizer contained in the remifentanil injection solution formulation (II) departs from the above-described range and the content ratio of the stabilizer is too low, it is difficult to obtain the remifentanil injection solution formulation in which the remifentanil (salts) has stably dissolved in the ethanol. On the other hand, in a case where the content ratio between the ethanol and the stabilizer contained in the remifentanil injection solution formulation (II) departs from the above-described range and the content ratio of the stabilizer is too high, an osmotic pressure is liable to become high.

It is favorable for the remifentanil injection solution formulation (I) and the remifentanil injection solution formulation (II) to contain 1 to 10mg of the remifentanil (salts) and more favorably to contain 2 to 5mg thereof per 1mL of the remifentanil injection solution formulation.

In a case where the content of the remifentanil (salts) per 1mL of the remifentanil injection solution formulation is a predetermined amount (for example, 1mg, 2mg or 5mg) falling within the above-described range, it is possible to easily prepare the remifentanil injection solution, having a predetermined concentration of the remifentanil, which can be used as it is at an anesthetizing time by taking out a whole amount of the remifentanil injection solution formulation from containers such as a vial, an ample or a prefilled syringe and mixing the remifentanil injection solution formulation with a predetermined amount of a diluting solution.

In a case where the remifentanil injection formulation of the present invention is the liquid remifentanil injection solution formulation, the remifentanil injection solution formulation may contain only the remifentanil (salts), the cyanocobalamin, the water, and the stabilizer or may contain only the remifentanil (salts), the cyanocobalamin, the ethanol, the stabilizer or may further contain other components depending on a case.

In a case where the remifentanil injection formulation of the present invention is the remifentanil injection solution formulation (I), it may additionally contain the ethanol in an amount not departing from the scope of the present invention. In a case where the remifentanil injection formulation of the present invention is the remifentanil injection solution formulation (II), it may additionally contain the water in an amount not departing from the scope of the present invention.

In a case where the remifentanil injection solution formulation of the present invention is the dry and solid formulation, it may contain only the remifentanil (salts) and the colorant cyanocobalamin or may contain the remifentanil (salts), the colorant cyanocobalamin, and other components.

The remifentanil injection solution formulation of the present invention can be stored, distributed, and sold by accommodating it inside a vial, an ample, a syringe, and the like. The material of the container is not specifically limited, but may be formed of any of glass, organic polymer such as plastic, rubber (elastomer), and composites of these materials. In a case where the container formed of the organic polymer is used, the container can be formed of the organic polymer such as polypropylene, cyclic olefin polymer, the rubber (elastomer), and the like.

The size of the container accommodating the remifentanil injection solution formulation is not specifically limited, but can be set to an appropriate size. For example, it is possible to set the size of the container to 0.2 to 20mL.

The capacity of the remifentanil injection solution formulation to be accommodated inside the container is not specifically limited, but an appropriate amount thereof can be accommodated therein. The remifentanil injection solution formulation may be accommodated inside each container in such a way that the amount of the remifentanil to be contained in one container is 1 to 10mg and favorably 2mg or 5mg.

The method for producing the remifentanil injection formulation of the present invention is not specifically limited.

In a case where the remifentanil injection formulation of the present invention is the liquid remifentanil injection solution formulation, it is possible to adopt a method capable of producing the remifentanil injection solution formulation (I) in which the remifentanil (salts), the cyanocobalamin, the water, the stabilizer, and other components used depending on a case are uniformly mixed with one another with the remifentanil (salts) and the cyanocobalamin dissolving in the water or a method capable of producing the remifentanil injection solution formulation (II) in which the remifentanil (salts), the cyanocobalamin, the ethanol, the stabilizer, and other components used depending on a case are uniformly mixed with one another with the remifentanil (salts) and the cyanocobalamin dissolving in the ethanol.

Although the method for producing the liquid remifentanil injection formulation (remifentanil injection solution formulation) of the present invention is not specifically limited, it is possible to obtain the liquid remifentanil injection formulation by mixing the remifentanil (salts), the cyanocobalamin, the water, the stabilizer, and other components used depending on a case with one another at 10 to 80 degrees C and favorably 20 to 50 degrees C to form a solution in which the remifentanil (salts) and the colorant (cyanocobalamin) have uniformly dissolved in the water or obtain the liquid remifentanil injection formulation by mixing the remifentanil (salts), the cyanocobalamin, the ethanol, the stabilizer, and other components used depending on a case with one another at 10 to 80 degrees C and favorably 20 to 50 degrees C to form a solution in which the remifentanil (salts) and the colorant (cyanocobalamin) have uniformly dissolved in the ethanol, thereafter aseptically filtering the solution, filling a predetermined amount of the solution in a container, and subjecting the solution to high-pressure steam sterilization. The aseptic filtration and the high-pressure steam sterilization can be performed by using methods similar to those conventionally adopted in producing the injection solution formulation.

In a case where the remifentanil injection formulation of the present invention is the dry and solid remifentanil injection formulation, it is possible to adopt both a method capable of producing the dry and solid remifentanil injection formulation in which the remifentanil (salts) and the colorant cyanocobalamin are uniformly mixed with one another and a method capable of producing the dry and solid remifentanil injection solution formulation in which the remifentanil (salts), the colorant selected from among the cyanocobalamin, the indocyanine green, and indigocarmine, and other components used depending on a case are uniformly mixed with one another.

Although the method for producing the dry and solid remifentanil injection formulation of the present invention is not specifically limited, it is possible to produce the granular or powdery dry and solid remifentanil injection formulation by mixing the remifentanil (salts), the colorant cyanocobalamin, the water, and other components used depending on a case with one another to prepare an aqueous solution containing the remifentanil (salts) and the colorant, thereafter aseptically filtering the aqueous solution, and filling a predetermined amount of the aqueous solution in a container or lyophilizing the predetermined amount of the aqueous solution without filling the aqueous solution in the container.

In preparing the final liquid remifentanil injection solution to be used at an anesthetizing time by using the remifentanil injection formulation of the present invention, it is possible to easily obtain the final remifentanil injection solution which correctly contains a predetermined concentration of the remifentanil (salts) by taking out a whole amount or a predetermined amount of the remifentanil injection solution formulation from a container with an appropriate means (for example, syringe, dropper, container connectable with syringe) and mixing the remifentanil injection solution formulation with a predetermined amount of the diluting solution (for example, saline, 5% glucose solution) to dilute the remifentanil injection solution formulation therewith.

In preparing the dry and solid remifentanil injection formulation to be used at an anesthetizing time by using the remifentanil injection formulation of the present invention, it is possible to easily obtain the dry and solid remifentanil injection solution which correctly contains a predetermined concentration of the remifentanil (salts) by adding a small amount of the dissolving solution to the container accommodating the remifentanil injection formulation to dissolve the remifentanil injection formulation therewith, taking out a whole amount or a predetermined amount of the remifentanil injection formulation from the container by using an appropriate means (for example, syringe, dropper, container connectable with syringe) and mixing the remifentanil injection formulation with a predetermined amount of the diluting solution (for example, saline, 5% glucose solution) to dilute the remifentanil injection formulation therewith.

The present invention includes a kit formulation having a container accommodating the remifentanil injection formulation of the present invention, a container accommodating the dissolving solution and/or a container accommodating the diluting solution. As the dissolving solution to be used for the kit formulation, it is possible to use water, saline, a 5% glucose injection solution, and the like. As the diluting solution to be used therefor, it is possible to use the saline, the 5% glucose injection solution, and the like.

In a case where the remifentanil injection formulation of the present invention is the liquid remifentanil injection solution formulation, the kit formulation having a container accommodating the remifentanil injection solution formulation and a container accommodating the diluting solution is exemplified.

In a case where the remifentanil injection formulation of the present invention is the dry and solid remifentanil injection solution formulation, the kit formulation having a container accommodating the solid remifentanil injection formulation, the container accommodating the dissolving solution, and the container accommodating the diluting solution and a kit formulation (a part of the diluting solution is used as the dissolving solution) having the container accommodating the solid remifentanil injection formulation and the container accommodating the diluting solution are exemplified.

The use of the kit formulation does not necessitate medical workers such as an anesthetist to take time and effort required to separately prepare or procure the dissolving solution or the diluting solution in addition to the remifentanil injection formulation. By using the kit formulation of the present invention, it is possible to easily prepare the colored remifentanil injection solution for anesthesia which allows medical workers to distinctly distinguish the remifentanil injection solution from the colorless dissolving and diluting solutions which have not been mixed with the remifentanil injection solution and do not contain the remifentanil.

### EXAMPLES

The present invention is specifically described below by using examples. Only examples 1 and 2 form part of the invention. But the present invention is not limited by the following examples.

### <Example 1>

### [Production of remifentanil injection solution formulation containing cyanocobalamin]

(1) 2.20mg of remifentanil hydrochloride (remifentanil: 2.00mg), 0.20mg of cyanocobalamin, 1.10g (0.98mL; specific gravity: 1.12g/mL) of macrogol 400 (polyethylene glycol 400) (stabilizer), and 0.02mL of water were mixed with one another at 20 degrees C to prepare a solution (concentration of remifentanil = 2mg/mL) containing 0.1mg of the cyanocobalamin per 1mg of the remifentanil and 2mg of the remifentanil per 1mL of the solution.
(2) After the solution obtained in the above (1) was aseptically filtered, 1mL of the remifentanil solution was filled in each container (syringe in which gasket was made of butyl rubber and syringe body was made of cyclic polyolefin) having a capacity of 1mL. Thereafter the containers were subjected to high-pressure steam sterilization. In this manner, a formulation for prefilled syringes in each of which a remifentanil injection solution containing the cyanocobalamin was filled was produced.

### <Example 2>

### [Production of lyophilized remifentanil injection formulation containing cyanocobalamin]

(1) 2.20mg of the remifentanil hydrochloride (remifentanil: 2.00mg) which was same as that used in the example 1, 15.00mg of glycine (excipient), 0.20mg of the cyanocobalamin which was same as that used in the example 1, and 1.00mL of water were mixed with one another at 20 degrees C to prepare a remifentanil solution.
(2) After the remifentanil solution obtained in the above (1) was aseptically filtered, 1mL of the remifentanil solution was filled in each container (vial container whose lid was made of rubber and whose body was made of glass) having a capacity of 1mL. Thereafter the containers were lyophilized for 14 hours with temperature being raised from -45 degrees C to 25 degrees C to produce a powdery remifentanil injection formulation containing the cyanocobalamin.

### <Example 3>

### [Production of lyophilized remifentanil injection formulation containing indigocarmine]

Except that 0.10mg of indigocarmine was used instead of 0.20mg of the cyanocobalamin used in (1) of the example 2, the same operation as that performed in (1) and (2) of the example 2 was performed to produce a powdery remifentanil injection formulation (lyophilized formulation) containing the indigocarmine.

### <Example 4>

### [Production of lyophilized remifentanil injection formulation containing indocyanine green]

Except that 0.20mg of indocyanine green was used instead of 0.20mg of the cyanocobalamin used in (1) of the example 2, the same operation as that performed in (1) and (2) of the example 2 was performed to produce a powdery remifentanil injection formulation (lyophilized formulation) containing the indocyanine green.

### <Reference Example 1>

### [Production of remifentanil injection solution formulation containing indigocarmine]

Except that 0.10mg of the indigocarmine which was same as that used in the example 3 was used instead of 0.20mg of the cyanocobalamin used in (1) of the example 1, the same operation as that performed in (1) and (2) of the example 1 was performed to produce a remifentanil injection solution formulation containing the indigocarmine.

### <Reference Example 2>

### [Production of remifentanil injection solution formulation containing indocyanine green]

Except that 0.20mg of the indocyanine green which was same as that used in the example 4 was used instead of 0.20mg of the cyanocobalamin used in (1) of the example 1, the same operation as that performed in (1) and (2) of the example 1 was performed to produce a remifentanil injection solution formulation containing the indocyanine green.

### <Comparison Example 1>

### [Production of lyophilized remifentanil injection formulation containing riboflavin phosphate ester sodium]

Except that 0.40mg of riboflavin phosphate ester sodium was used instead of 0.20mg of the cyanocobalamin used in (1) of the example 2, the same operation as that performed in (1) and (2) of the example 2 was performed to produce a powdery remifentanil injection formulation (lyophilized formulation) containing the riboflavin phosphate ester sodium.

### <Comparison Example 2>

### [Production of remifentanil injection solution formulation containing riboflavin phosphate ester sodium]

Except that 0.40mg of the riboflavin phosphate ester sodium which was the same as that used in the comparison example 1 was used instead of 0.20mg of the cyanocobalamin used in (1) of the example 1, the same operation as that performed in (1) and (2) of the example 1 was performed to produce a remifentanil injection solution formulation containing the riboflavin phosphate ester sodium.

The reason the content of the riboflavin phosphate ester sodium contained in the remifentanil injection formulation was set to 0.40mg in the comparison examples 1 and 2 is because if the content of the riboflavin phosphate ester sodium is 0.20mg, the remifentanil injection solution for anesthesia obtained by dissolving and diluting the remifentanil injection formulation by using dissolving and diluting solutions is colored so thinly that it is visually difficult to check whether the injection solution was obtained by mixing the remifentanil injection solution formulation with the riboflavin phosphate ester sodium.

### <Comparison Example 3>

### [Production of lyophilized remifentanil injection formulation containing uranine]

Except that 1.00mg of uranine was used instead of 0.20mg of the cyanocobalamin used in (1) of the example 2, the same operation as that performed in (1) and (2) of the example 2 was performed to produce a powdery remifentanil injection formulation (lyophilized formulation) containing the uranine.

### <Comparison Example 4>

### [Production of remifentanil injection solution formulation containing uranine]

Except that 1.00mg of the uranine which was the same as that used in the comparison example 3 was used instead of 0.20mg of the cyanocobalamin used in (1) of the example 1, the same operation as that performed in (1) and (2) of the example 1 was performed to produce a remifentanil injection solution formulation containing the uranine.

The reason the content of the uranine contained in the remifentanil injection formulation was set to 1.00mg in the comparison examples 3 and 4 is because if the content of the uranine is less than 1.00mg, the remifentanil injection solution for anesthesia obtained by dissolving and diluting the remifentanil injection formulation by using dissolving and diluting solutions is colored so thinly that it is visually difficult to check whether the injection solution was obtained by mixing the remifentanil injection solution formulation with the uranine.

### [Stability Test 1]

The remifentanil injection formulation obtained in each of the above-described examples 1 through 4, the reference examples 1 and 2, and the comparison examples 1 through 4 was stored at 60 degrees C for three weeks. The amount of the remifentanil in each solution was determined when the test started and when three weeks passed after the test finished. The survival rate (%) of the remifentanil when three weeks passed was determined with respect to the survival rate set to 100% when the test started. In each example, reference example and comparison example, the survival rate of the remifentanil was determined by using three specimens, conducting the same test(n=3), and taking the average(%) of three values.

The results are shown in table 1.

### [Quantitative Method]

(1) 1mL of the remifentanil injection solution formulation obtained in each of the example 1, the reference examples 1 and 2, and the comparison examples 2 and 4 was correctly measured. Saline was added to the granular lyophilized formulation of each of the examples 2 through 4 and the comparison examples 1 and 3 to dissolve each lyophilized formulation to obtain solutions each having a volume of 10mL correctly. After 4mL of each solution was correctly measured, 8mL of an internal standard solution [mobile phase solution (1 → 40000) of methyl parahydroxybenzoate] was added to each of the solutions having the volume of 4mL. Thereafter a mobile phase [mixed solution of phosphoric acid-based buffer solution (pH 2.5): acetonitrile = 78:22 (volume ratio)] was further added to each of the solutions to obtain specimen solutions each having a volume of 20mL.
(2) Separately from the above-described (1), after about 20mg of remifentanil hydrochloride was precisely measured, the mobile phase [mixed solution of phosphoric acid-based buffer solution (pH 2.5): acetonitrile = 78:22 (volume ratio)] was added to solutions of the remifentanil hydrochloride to obtain solutions each having a volume of exactly 100mL. After 4mL of each of these solutions was correctly measured, 8mL of the internal standard solution was correctly added to each of these solutions. Thereafter the mobile phase [mixed solution of phosphoric acid-based buffer solution (pH 2.5): acetonitrile = 78:22 (volume ratio)] was added to the solutions to obtain specimen solutions each having a volume of 20mL.
(3) By using 10µL of each of the specimen solutions prepared in the above-described (1) and 10µL of each of the specimen solutions prepared in the above-described (2), the amount of each remifentanil was measured by using a chromatographic method in conditions described below.

The peak area of each solution was measured by an automatic integration method to determine the amount of each remifentanil from the ratio of the peak area of the remifentanil to the peak area of the internal standard solution [mobile phase solution (1 → 40000) of methyl parahydroxybenzoate)].

### <Measuring Condition>

- Detector: ultraviolet absorption meter (measured wavelength: 210nm)
- Column: a stainless steel pipe having an inner diameter of 4.6mm and a length of 25cm in which 5µm of octadecylsilyl silica gel for use in a liquid chromatograph was filled
- Temperature of column: constant in the vicinity of 40 degrees C
- Mobile phase: obtained by dissolving 2.9g of ammonium dihydrogen phosphate in 1000mL of water and adding phosphoric acid to the obtained solution to adjust the pH thereof to about 2.5 and adding 220mL of acetonitrile to 780mL of the solution.
- Internal standard solution: mobile phase solution of methyl parahydroxybenzoate (1 to 40000)
- Flow rate: The holding period of time of the peak of the remifentanil was set to about 10 minutes.

**Table 1**

| | Details of remifentanil injection formulation | | | | Stability of remifentanil injection formulation | |
|---|---|---|---|---|---|---|
| | Content of remifentanil | Colorant | | Form | Survival rate after storage of 3 weeks at 60 degrees C | Color change and fading during storage of 3 weeks at 60 degrees C |
| | | Kind | Content | | | |
| Example 1 | 2.00 mg | Cyanoco balamin | 0.20mg | Liquid | 95% | No color change and fading |
| Example 2 | 2.00 mg | Cyanoco balamin | 0.20mg | Powder | 97% | No color change and fading |
| Example 3 | 2.00 mg | Indigo carmine | 0.10mg | Powder | 97% | No color change and fading |
| Example 4 | 2.00 mg | Indocyanine green | 0.20mg | Powder | 97% | No color change and fading |
| Reference example 1 | 2.00 mg | Indigo carmine | 0.10mg | Liquid | 95% | Faded |
| Reference example 2 | 2.00 mg | Indocyanine green | 0.20mg | Liquid | 94% | Changed |
| Comparison example 1 | 2.00 mg | Riboflavin phosphate ester sodium | 0.40mg | Powder | 75% | No color change and fading |
| Comparison example 2 | 2.00 mg | Riboflavin phosphate ester sodium | 0.40mg | Liquid | 75% | No color change and fading |
| Comparison example 3 | 2.00 mg | Uranine | 1.00mg | Powder | 55% | No color change and fading |
| Comparison example 4 | 2.00 mg | Uranine | 1.00mg | Liquid | 55% | No color change and fading |

As shown in the table 1, the remifentanil injection formulation of each of the examples 1 through 4 containing the colorant selected from among the cyanocobalamin, the indocyanine green, and the indigocarmine had a survival rate as high as not less than 95% after the remifentanil injection formulations were stored at 60 degrees C for three weeks (corresponding to storage for three years at room temperature), which indicates that these remifentanil injection formulations were excellent in the long-term storage stability thereof at the room temperature.

On the other hand, the remifentanil injection formulation of each of the comparison examples 1 through 4 containing the riboflavin phosphate ester sodium or the uranine had a survival rate as low as 75% or 55% after the remifentanil injection formulations were stored at 60 degrees C for three weeks, which indicates that these remifentanil injection formulations had a low storage stability and did not have a practical value.

### INDUSTRIAL APPLICABILITY

The remifentanil injection formulation of the present invention containing the colorant selected from among the cyanocobalamin, the indocyanine green, and the indigocarmine is excellent it its long-term storage stability at the room temperature. The remifentanil injection formulation of the present invention is colored when it is dissolved and diluted by the dissolving and diluting solutions to prepare the remifentanil injection solution to be used at an anesthetizing time. Thus, the remifentanil injection solution can be distinctly distinguished from the colorless dissolving and diluting solutions. Therefore, the remifentanil injection formulation of the present invention is very useful because at an anesthesia place, it is possible to reliably prevent the occurrence of medical malpractice of erroneously injecting the dissolving and diluting solutions which have not been mixed with the remifentanil injection formulation and do not contain the remifentanil to patients.

## Claims

1. A remifentanil injection formulation containing remifentanil and/or physiologically acceptable salts of said remifentanil and a colorant, **characterized in that** said colorant is cyanocobalamin.

2. A remifentanil injection formulation according to claim 1, wherein said colorant is contained at a rate of 0.02 to 0.30mg per 1mg of said remifentanil.

3. A remifentanil injection formulation according to claim 1 or 2, which is a liquid remifentanil injection solution formulation.

4. A remifentanil injection formulation according to claim 3, wherein said remifentanil injection solution formulation contains said remifentanil and/or said physiologically acceptable salts of said remifentanil as said remifentanil at a rate of 1 to 10mg in 1mL of said remifentanil injection solution formulation.

5. A remifentanil injection formulation according to claim 1 or 2, which is dry.

6. A remifentanil injection formulation according to any one of claims 1 to 4, wherein said remifentanil injection formulation contains a stabilizer, and said stabilizer is a physiologically acceptable liquid polyhydric alcohol.

7. A kit formulation comprising a container accommodating a remifentanil injection formulation according to any one of claims 1 through 6 and a container accommodating a dissolving solution and/or a container accommodating a diluting solution.

## Patentansprüche

1. Remifentanil-Injektionsformulierung, die Remifentanil und/oder physiologisch akzeptable Salze des Remifentanils und einen Farbstoff enthält, **dadurch gekennzeichnet, dass** es sich bei dem Farbstoff um Cyanocobalamin handelt.

2. Remifentanil-Injektionsformulierung nach Anspruch 1, wobei der Farbstoff in einer Menge von 0,02 bis 0,30 mg pro 1 mg Remifentanil enthalten ist.

3. Remifentanil-Injektionsformulierung nach Anspruch 1 oder 2, bei der es sich um eine flüssige Remifentanil-Injektionslösungsformulierung handelt.

4. Remifentanil-Injektionsformulierung nach Anspruch 3, wobei die Remifentanil-Injektionslösungsformulierung das Remifentanil und/oder die physiologisch akzeptablen Salze des Remifentanils als Remifentanil in einer Menge von 1 bis 10 mg in 1 ml der Remifentanil-Injektionslösungsformulierung enthält.

5. Remifentanil-Injektionsformulierung nach Anspruch 1 oder 2, die trocken ist.

6. Remifentanil-Injektionsformulierung nach einem der Ansprüche 1 bis 4, wobei die Remifentanil-Injektionsformulierung einen Stabilisator enthält und der Stabilisator ein physiologisch akzeptabler flüssiger mehrwertiger Alkohol ist.

7. Kit-Formulierung mit einem Behälter, der eine Remifentanil-Injektionsformulierung nach einem der Ansprüche 1 bis 6 enthält, und einem Behälter, der eine Auflösungslösung enthält, und/oder einem Behälter, der eine Verdünnungslösung enthält.

## Revendications

1. Composition injectable de rémifentanil, contenant du rémifentanil et/ou un sel physiologiquement acceptable dudit rémifentanil et un colorant, **caractérisée en ce que** ledit colorant est la cyanocobalamine.

2. Composition injectable de rémifentanil selon la revendication 1, dans laquelle ledit colorant est contenu en une proportion de 0,02 à 0,30 mg par mg dudit rémifentanil.

3. Composition injectable de rémifentanil selon la revendication 1 ou 2, qui est une composition de solution injectable de rémifentanil liquide.

4. Composition injectable de rémifentanil selon la revendication 3, où ladite composition de solution injectable de rémifentanil contient ledit rémifentanil et/ou ledit sel physiologiquement acceptable dudit rémifentanil en tant que ledit rémifentanil en une proportion de 1 à 10 mg dans 1 mL de ladite composition de solution injectable de rémifentanil.

5. Composition injectable de rémifentanil selon la revendication 1 ou 2, qui est sèche.

6. Composition injectable de rémifentanil selon l'une quelconque des revendications 1 à 4, où ladite composition injectable de rémifentanil contient un stabilisant, et ledit stabilisant est un alcool polyhydrique liquide physiologiquement acceptable.

7. Composition de nécessaire comprenant un contenant recevant une composition injectable de rémifentanil selon l'une quelconque des revendications 1 à 6 et un contenant recevant une solution pour dissolution et/ou un contenant recevant une solution pour dilution.
